# EUROPEAN PATENT SPECIFICATION

(11) **EP 2 858 623 B1**
(45) Date of publication and mention of the grant of the patent: **28.09.2016**
(21) Application number: 13742275.4
(22) Date of filing: 05.06.2013
(51) Int. Cl.: A61K 8/49, A61Q 19/00

(54) **ACTIVE COMPOUNDS FOR COMBATING GREASY SKIN**
AKTIVE VERBINDUNGEN ZUR BEKÄMPFUNG VON FETTIGER HAUT
COMPOSÉS ACTIFS POUR COMBATTRE LA PEAU GRASSE

(30) Priority: 06.06.2012 FR 1255260; 06.06.2012 FR 1255269
(43) Date of publication of application: 15.04.2015
(73) Proprietor: L'Oréal, 75008 Paris (FR)
(72) Inventor: MARAT, Xavier, F-75010 Paris (FR); MULLER, Benoit, F-75019 Paris (FR); THOMAS-COLLIGNON, Agnés, F-78180 Montigny le Bretonneux (FR); BERNARD, Dominique, F-92170 Vanves (FR)
(74) Representative: Nony
(86) International application number: PCT/IB2013/054634
(87) International publication number: WO 2013/183016

(56) References cited:
- WO-A1-83/02891
- WO-A1-2012/077034
- WO-A2-2008/008704
- US-A- 4 211 767
- MADHURI MANPADI ET AL: "Three-component synthesis and anticancer evaluation of polycyclic indenopyridines lead to the discovery of a novel indenoheterocycle with potent apoptosis inducing properties", ORGANIC & BIOMOLECULAR CHEMISTRY, vol. 5, no. 23, 2007, page 3865, XP055054159, ISSN: 1477-0520, DOI: 10.1039/b713820b
- NIKOLAI M. EVDOKIMOV ET AL: "Structural Simplification of Bioactive Natural Products with Multicomponent Synthesis. 3. Fused Uracil-Containing Heterocycles as Novel Topoisomerase-Targeting Agents", JOURNAL OF MEDICINAL CHEMISTRY, vol. 54, no. 7, 14 April 2011 (2011-04-14) , pages 2012-2021, XP055054137, ISSN: 0022-2623, DOI: 10.1021/jm1009428

## Description

The present invention aims to provide novel compounds for preventing and/or treating a disorder, in particular an aesthetic disorder, of the skin. In particular, the present invention relates to disorders associated with greasy skin.

The term "the skin" means the entire skin of the body, including the scalp and mucous membranes. The term "skin integuments" means bodily hairs, the eyelashes, head hair and the nails.

The skin, or epidermis, is conventionally divided into a basal layer of keratinocytes that constitutes the germinal layer of the epidermis, a spinous layer consisting of several layers of polyhedral cells positioned on the germinal layer, one to three "granular" layers consisting of flattened cells containing distinct cytoplasmic inclusions, keratohyalin granules, and finally an assembly of upper layers known as cornified layers, or *stratum corneum*, consisting of keratinocytes at the terminal stage of their differentiation, known as corneocytes.

Many aesthetic disorders or pathological defects of the skin or its integuments may be associated with an epidermal homeostasis defect and in particular a cell proliferation and/or differentiation defect.

Sebum is normally an agent which moisturizes the epidermis and may be involved in homeostasis of the epidermis. It is the natural product of the sebaceous gland, which is an annex of the pilosebaceous unit. It is essentially a more or less complex mixture of lipids. Conventionally, the sebaceous gland produces squalene, triglycerides, aliphatic waxes, cholesterol waxes and, possibly, free cholesterol. The action of bacterial lipases converts a variable proportion of the triglycerides formed into free fatty acids. The sebocyte constitutes the competent cell of the sebaceous gland. The production of sebum is associated with a programme of terminal differentiation of this cell. During this differentiation, the metabolic activity of the sebocyte is essentially directed towards the biosynthesis of lipids *(lipogenesis)* and more precisely towards fatty acid neosynthesis.

Hyperseborrhoeic or greasy skin is especially characterized by an exaggerated secretion and excretion of sebum. Conventionally, a sebum level of greater than 200 µg/cm² measured on the forehead is considered as being characteristic of such greasy skin. Such skin is also often associated with a desquamation defect, a shiny complexion, a thick skin grain or dilated pores, which manifestations are perceived as being skin imperfections or aesthetic defects.

Besides its unsightly appearance, greasy skin or greasy-prone skin is a terrain on which may arise complications affecting zones in which the sebaceous glands are numerous, participating especially in the appearance of common acne lesions, dermatitis or pruritus.

Thus, skin disorders associated with greasy skin are one of the major targets of cosmetics and dermatology. These disorders particularly affect large populations such as adolescents or, more globally, Asiatic populations. Despite substantial research efforts in this field, satisfactory novel technical solutions still remain to be provided and novel targets and active agents acting on these targets still remain to be discovered. Thus, to combat hyperseborrhoea, the prior art has already proposed various compounds, which, by topical application to the skin, are capable of reducing the lipogenesis of the sebocytes and consequently of limiting the production of sebum. The treatments currently available for hyperseborrhoea are not entirely satisfactory, especially with regard to the side effects that are frequently associated therewith, such as irritant effects with certain topical agents such as retinoids and benzoyl peroxides.

It is thus of major importance to discover novel targets and novel active compounds that act on the cause of greasy skin or greasy-prone skin, or associated skin disorders.

Moreover, it has been indicated, in the applications filed under numbers FR 10 601 83 and FR 10 601 79, that the interaction between SASPase and FLG2 leads to an increase in the proteolytic activity of SASPase. This proteolytic activity is involved in the degradation of corneodesmosin, and consequently participates in the regulation of epidermal homeostasis, in particular through the regulation of cell desquamation, proliferation or differentiation phenomena. Furthermore, this function of SASPase has been confirmed by the observation of its ability to hydrolyse certain forms of filaggrin, which is a protein essential to the organization, the function and the moisturization of the epidermal barrier (Matsui et al., EMBO Mol Med, 2011, 3:320).

Providing active compounds that are capable of modulating this interaction thus proves to be a major asset in the production of a cosmetic or therapeutic arsenal with regard to greasy skin or greasy-prone skin, or associated skin disorders.

There is thus still a need for novel active agents that are capable of exerting beneficial cosmetic or therapeutic action on greasy skin or greasy-prone skin and/or the associated cutaneous aesthetic defects or pathological disorders.

There is also still a need for active agents that can re-establish the homeostasis of the epidermis of greasy skin or greasy-prone skin.

There is also a need for novel compositions that are effective in preventing and/or treating greasy skin or greasy-prone skin and that are pleasant and comfortable to use, thus promoting adherence to the treatment.

There is also a need for novel active agents for preventing and/or treating pathological disorders of greasy skin, especially such as seborrhoeic dermatitis or acne.

There is also a need for novel active agents for moisturizing greasy skin or greasy-prone skin, and for reinforcing the barrier function properties of the skin.

There is also a need for novel active agents that are capable of exerting beneficial cosmetic or therapeutic action on greasy scalp conditions.

The object of the present invention is to satisfy these needs.

Thus, according to a first subject, the invention relates to the cosmetic use, as an agent for preventing and/or treating greasy skin or greasy-prone skin and/or an associated aesthetic skin disorder, of an effective amount of at least one compound represented by either of the general formulae (Ia) and (Ib): 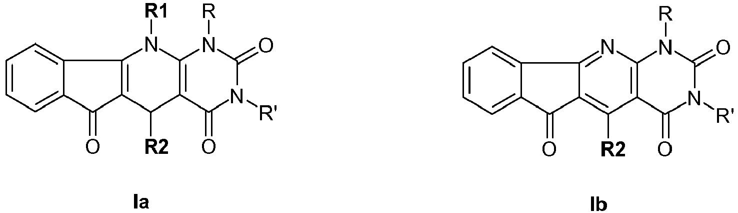 in which:
- R¹ represents H or a group -C(O)R⁸, with R⁸ representing a linear or branched, saturated or unsaturated C₁-C₄, or even C₂-C₃, alkyl, or being a phenyl;
- R² represents H; a linear or branched, saturated or unsaturated C₁-C₁₂, in particular C₁-C₆ or even C₂-C₄ alkyl group; or a group chosen from: 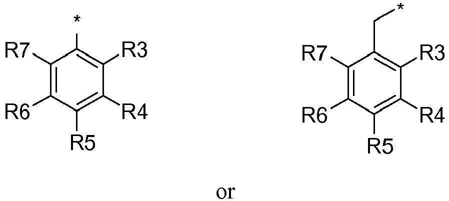 in which R³, R⁴, R⁵, R⁶ and R⁷ represent, independently of each other, H; -NO₂; -OH; -OR⁸; -CN; Br; Cl; I; F; -CF₃; a linear or branched, saturated or unsaturated C₁-C₄, or even C₂-C₃, alkyl; a phenyl; -OC(O)-R⁸; -O-Ph-X with X representing H, -OH, -NO₂, a fluorine, a linear or branched, saturated or unsaturated C₁-C₄, or even C₂-C₃, alkyl or alkoxy; R⁸ being as defined previously,
- R and R', which may be identical or different, represent H or a linear or branched C₁-C₄, or even C₂-C₃, alkyl,
or a physiologically acceptable salt thereof.

The inventors have observed, unexpectedly, that such compounds make it possible to induce and stimulate accelerated formation of a thickened *stratum corneum*, have strong anti-proliferative properties, modify the proliferation/differentiation balance of skin cells towards a reduction of the live layers and an increase of the cornified layers, and/or promote the maturation of the *stratum corneum* (SC) and/or the increase of layers of the SC. All these properties thus make the compounds of the invention particularly effective with regard to greasy skin or greasy-prone skin, and associated skin disorders.

The use of a compound of the invention advantageously makes it possible to regulate the production of sebum efficiently while at the same time ensuring good tolerance.

According to one advantage, a use according to the invention may make it possible to prevent and/or treat an aesthetic disorder chosen from skin imperfections caused by hyperseborrhoea, and/or a scalp disorder.

According to another advantage, a use according to the invention can reduce and/or treat the pruritus of greasy skin or greasy-prone skin.

According to another advantage, a use according to the invention can promote moisturization and maintenance of the integrity of greasy skin or greasy-prone skin.

According to another of its aspects, the present invention relates to a compound of the invention, in an effective amount in a pharmaceutical or dermatological composition, for preventing and/or treating a pathological disorder of greasy skin or greasy-prone skin.

In particular, such a composition proves to be effective for preventing and/or treating a pathological disorder chosen from seborrhoeic dermatitis, acne and seborrhoeic pruritus.

According to another of its aspects, the present invention relates to a cosmetic process for preventing and/or treating greasy skin or greasy-prone skin and/or an associated aesthetic skin disorder, in an individual in need thereof, comprising at least one step of administering, to the said individual, at least an effective amount of at least one compound of the invention.

According to another of its aspects, the invention relates to a compound, as such, represented by the general formula (IIa): 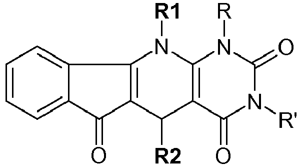 IIa in which:
- R¹ represents H, and
- R² represents a linear or branched, saturated C₁-C₄ alkyl, preferably a C₁-C₃ alkyl, or even a C₂-C₃ alkyl, a benzyl, or 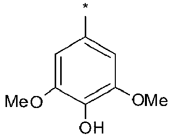
- R and R' represent a methyl,
or a physiologically acceptable salt thereof.

According to another of its aspects, the present invention relates to a cosmetic or dermatological composition comprising, in a physiologically acceptable medium, an efficient amount of at least one compound represented by the general formula (IIa) as defined above, and more particularly as defined here after.

For the purposes of the present invention, the term "preventing" means reducing a risk of manifestation or reducing a probability of occurrence of the phenomenon in question.

For the purposes of the present invention, the term "effective amount" of a compound of the invention means an amount of this compound that is sufficient and necessary to exert a preventive and/or treating effect with regard to greasy skin or greasy-prone skin and/or associated aesthetic or pathological skin disorders. Such an amount can be determined by any method known to a person skilled in the art, for example by means of *in vitro, ex vivo* or *in vivo* trials, such as clinical trials.

For the purposes of the present invention, the term "physiologically or cosmeceutically or pharmaceutically acceptable medium" means a medium that is compatible with administration to an individual, especially onto or into the skin and/or the integuments of this individual.

For the purposes of the present invention, an alkyl or alkoxy of a certain length encompasses all individual sub-ranges of alkyl or alkoxy respectively. Thus, and in a non-limitative way, a C₁-C₁₂ alkyl or alkoxy encompasses a C₁-C₁₂; a C₁-C₁₁; a C₁-C₁₀; a C₁-C₉; a C₁-C₈; a C₁-C₇; a C₁-C₆; a C₁-C₅; a C₁-C₄; a C₁-C₃; a C₁-C₂ ; a C₂-C₄; or a C₂-C₃ alkyl or alkoxy.

Accordingly, a C₁-C₄ alkyl or alkoxy encompasses a C₁-C₄; a C₁-C₃; a C₁-C₂; a C₂-C₄; a C₂-C₃ or a C₃-C₄ alkyl or alkoxy.

### Greasy skin

A compound of the invention may advantageously be used for treating and/or preventing greasy skin or greasy-prone skin and/or the associated cutaneous signs.

Depending on the degree of intensity of the manifestation of the disorder associated with greasy skin, the said disorder may be a matter of an aesthetic disorder or a pathological disorder of greasy skin. Such a classification falls within the competence of those skilled in the art.

The invention is directed towards all bodily skin, including the scalp, and, preferably, the skin of the face, neckline, neck, arms and forearms, or even, more preferably, the skin of the face, neckline and neck.

According to one embodiment, an aesthetic disorder of greasy skin or greasy-prone skin may be chosen from skin imperfections caused by hyperseborrhoea and/or a scalp disorder.

The aesthetic cutaneous signs of hyperseborrhoea, or greasy skin, which are more particularly considered by the invention may be shiny and/or thick skin and/or skin whose follicular orifices or pores are dilated, or even, in certain cases, are filled with tiny cornified spicules or with comedones. Greasy skin is often associated with a desquamation defect and with a thick skin grain.

The aesthetic cutaneous disorders, or imperfections, of greasy skin or greasy-prone skin may be chosen in particular from a thick skin grain, shiny skin, skin with dilated pores or follicular orifices, skin with pores or follicular orifices filled with cornified spicules or with comedones, dyschromia, redness, rough skin with, where appropriate, dry skin plaques, or skin with an impaired complexion.

Greasy skin or greasy-prone skin may also show impairments in the skin's complexion, such as a sallow, non-uniform, dull, waxy or sickly complexion.

According to another embodiment, a pathological skin disorder associated with greasy skin or greasy-prone skin may be chosen from seborrhoeic dermatitis, acne and seborrhoeic pruritus.

In their pathological manifestations, the cutaneous signs of hyperseborrhoea may appear in the form of seborrhoeic dermatitis, seborrhoeic pruritus, comedones and/or acne lesions. Acne lesions are manifested in the form of pimples which finish by drying up.

Acne is a multi-factor disease of the follicle of the sebaceous gland that attacks skin rich in sebaceous glands (face, shoulder area, arms and intertriginous areas). Acne is the main one of the most common forms of dermatosis. In its mildest form, this dermatosis affects almost all human beings. Its frequency is maximal at the age of puberty, but it may appear for the first time from the age of 7 to 9 and may extend beyond the age of 40. It also affects both men and women.

Among its commonest forms, mention may be made of comedonal acne, commonly referred to as juvenile acne, papulo-pustular and/or nodular acne, acne conglobata and "exogenous" acne appearing in reaction to inflammatory external factors.

The clinical manifestations of acne are known as retention lesions and may be of the type such as open or closed comedones, for instance microcysts, microcomedones or whiteheads. The inflammatory lesions derived from the retention lesions may be of the type such as papules, pustules, with indurated nodules, abscesses, fistulae or scar forms.

Acneic or acne-prone individuals usually have greasy skin, greasy-prone skin or combination skin. Their skin is usually shiny with numerous imperfections on the face, inter alia, such as microcysts, microcomedones, whiteheads, papules, pustules, with indurated nodules, abscesses, fistulae or scar forms. The imperfections may also be of the type such as dull, sallow skin, dyschromia, redness, or rough skin with dry skin plaques. Cutaneous hyperkeratosis is observed, the facial pores are dilated, the skin is often rough with a thick *stratum corneum* revealing in plaques areas of dry skin resulting from epidermal atrophy and mild desquamation.

A scalp disorder may appear, depending on its severity, in the form of an irritation or a seborrhoeic dermatitis, accompanied, where appropriate, by irritation and/or itching of the scalp.

The invention may prove to be most particularly useful:
- for preventing and/or treating the aesthetic defects of greasy skin or greasy-prone skin,
- for preventing and/or treating seborrhoeic dermatitis of the skin,
- for preventing and/or treating skin with dilated pores or follicular orifices, skin with pores or follicular orifices filled with cornified spicules or with comedones, dyschromia, redness, rough skin with, where appropriate, dry skin plaques, or skin with an impaired complexion,
- for preventing and/or treating an impaired skin complexion,
- for preventing and/or treating an aesthetic defect of the scalp associated with excessive excretion and/or secretion of sebum,
- for improving the comfort of the skin or the scalp, or
- for preserving and/or reinforcing the integrity of the barrier functions of the skin or the scalp.

### Compounds

According to one embodiment, a compound that is more particularly under consideration according to the invention may be represented by the general formula (Ia) or (Ib) as defined previously.

According to a preferred embodiment, a compound of the invention may be represented by the general formula (Ia), in which:
- R¹ represents H,
- R² represents H; a linear or branched, saturated or unsaturated C₁-C₁₂, in particular C₁-C₆, or even C₂-C₄, alkyl group; or a group chosen from: 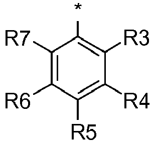 or 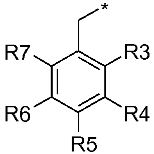 in which R³, R⁴, R⁵, R⁶ and R⁷ represent, independently of each other, H; -NO₂; -OH; -OR⁸; -CN; F; -CF₃; a linear or branched, saturated or unsaturated C₁-C₄, or even C₂-C₃, alkyl; a phenyl; -OC(O)-R⁸; -O-Ph-X with X representing H, -OH, -NO₂, a fluorine, a linear or branched, saturated or unsaturated C₁-C₄ alkyl or alkoxy; with R⁸ being a linear or branched, saturated or unsaturated C₁-C₄, or even C₂-C₃, alkyl, or being a phenyl,
- R and R', which may be identical or different, represent H or a linear or branched C₁-C₄, or even C₂-C₃, alkyl,
or a physiologically acceptable salt thereof.

According to another preferred embodiment, a compound of the invention may be represented by the general formula (Ia) in which:
- R¹ represents H,
- R² represents H; a linear or branched, saturated or unsaturated C₁-C₁₂, in particular C₁-C₆ or even C₂-C₄, alkyl group; or a group chosen from: 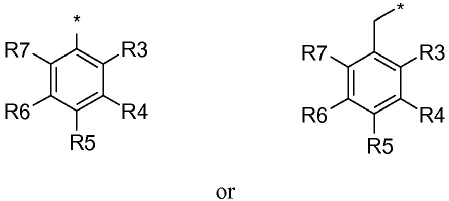 in which R³, R⁴, R⁵, R⁶ and R⁷ represent, independently of each other, H; -OH; -OR⁸; -CN; a linear or branched, saturated or unsaturated C₁-C₄, or even C₂-C₃, alkyl; a phenyl; -OC(O)-R⁸; -O-Ph-X with X representing H, -OH, a linear or branched, saturated or unsaturated C₁-C₄ alkyl or alkoxy; with R⁸ being a linear or branched, saturated or unsaturated C₁-C₄, or even C₂-C₃, alkyl, or being a phenyl,
- R and R', which may be identical or different, represent H or a methyl,
or a physiologically acceptable salt thereof.

According to yet another preferred embodiment, a compound of the invention may be represented by the general formula (Ia) in which:
- R¹ represents H,
- R² represents H, a linear or branched, saturated C₁-C₆, or even C₂-C₄, alkyl group; or a group chosen from: 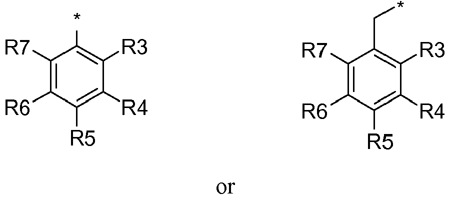 in which R³, R⁴, R⁵, R⁶ and R⁷ represent, independently of each other, H; -OH; -OR⁸; -CN; a linear or branched, saturated or unsaturated C₁-C₄, or even C₂-C₃, alkyl; with R⁸ being a linear or branched, saturated or unsaturated C₁-C₄, or even C₂-C₃, alkyl, or being a phenyl,
- R and R' represent a methyl,
or a physiologically acceptable salt thereof.

According to yet another preferred embodiment, a compound of the invention may be represented by the general formula (Ia) in which:
- R¹ represents H,
- R² represents a linear or branched, saturated or unsaturated C₁-C₄, or even C₂-C₃ alkyl group; or a group chosen from: 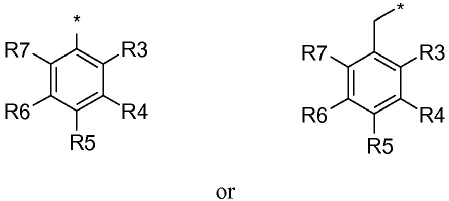 in which R³, R⁴, R⁵, R⁶ and R⁷ represent, independently of each other, H; -OH; -OR⁸; -CN; a linear or branched, saturated or unsaturated C₁-C₃ alkyl; a phenyl; with R⁸ being a linear or branched, saturated or unsaturated C₁-C₃ alkyl,
- R and R', which may be identical or different, represent H or a linear or branched, saturated or unsaturated C₁-C₃ alkyl,
or a physiologically acceptable salt thereof.

According to yet another preferred embodiment, a compound of the invention may be represented by the general formula (Ia) in which:
- R¹ represents H,
- R² represents a linear or branched, saturated C₁-C₄, or even C₂-C₃, alkyl group, and preferably represents a methyl, an ethyl, an n-propyl, an isopropyl, an n-butyl, a sec-butyl, an isobutyl or a *tert*-butyl, and preferentially an isobutyl; a group 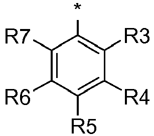 in which R³, R⁴, R⁵, R⁶ and R⁷ represent, independently of each other, H; -OH; -OR⁸; -CN, with R⁸ representing a linear or branched, saturated or unsaturated C₁-C₂ alkyl, and preferably a methyl; or a group 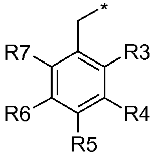 in which R³, R⁴, R⁵, R⁶ and R⁷ represent H,
- R and R', which may be identical or different, represent H or a linear or branched, saturated or unsaturated C₁-C₂ alkyl, and preferably a methyl,
or a physiologically acceptable salt thereof.

According to yet another preferred embodiment, a compound of the invention may be represented by the general formula (IIa) in which: 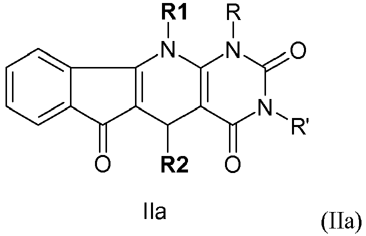 in which:
- R¹ represents H, and
- R² represents a linear or branched, saturated C₁-C₄ alkyl, preferably a C₁-C₃ alkyl, or even a C₂-C₄ or C₂-C₃ alkyl; a benzyl; or 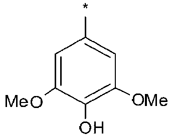
- R and R' represent a methyl,
or a physiologically acceptable salt thereof.

More preferably, R² may represent a group chosen from: 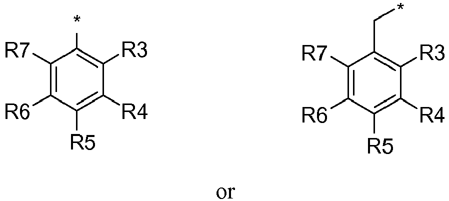 in which R³ and R⁷ represent H, and R⁴, R⁵ and R⁶ represent, independently of each other, a group as defined previously, and preferably H; -OH; -OR⁸; -CN, with R⁸ representing a linear or branched, saturated or unsaturated C₁-C₂ alkyl, and preferably a methyl.

According to yet another preferred embodiment, a compound of the invention may be chosen from: 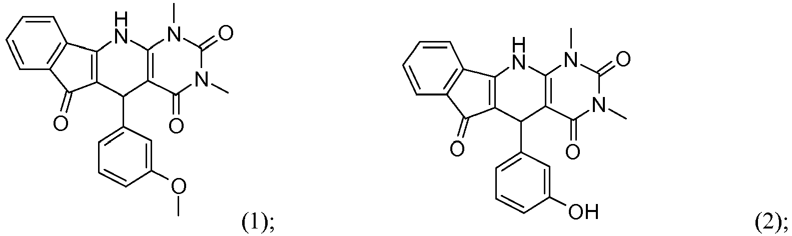 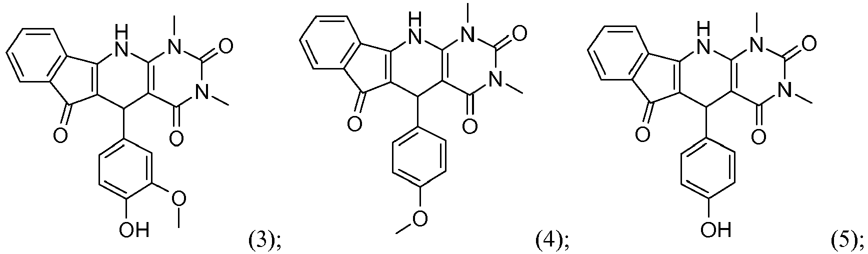 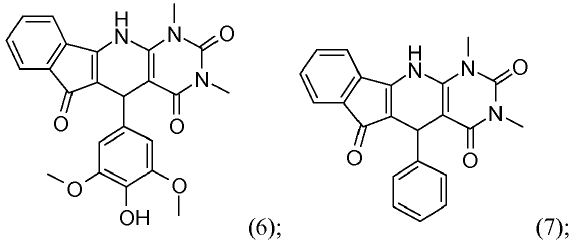 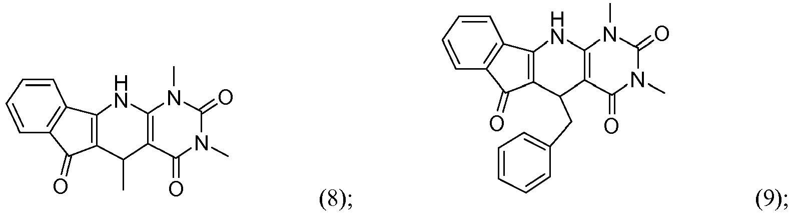 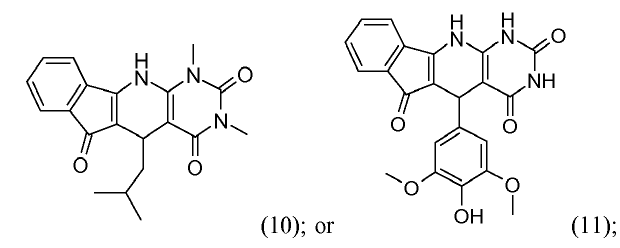 or a physiologically acceptable salt.

A subject of the invention is also, per se, a compound represented by the general formula (IIa): 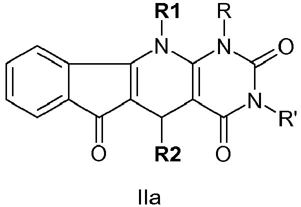 in which:
- R¹ represents H, and
- R² represents a linear or branched, saturated C₁-C₄ alkyl, preferably a C₁-C₃ alkyl, or even a C₂-C₄ or C₂-C₃ alkyl, a benzyl, or 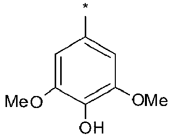
- R and R' represent a methyl,
or a physiologically acceptable salt thereof.

More preferably, a subject of the invention is, per se, a compound chosen from: 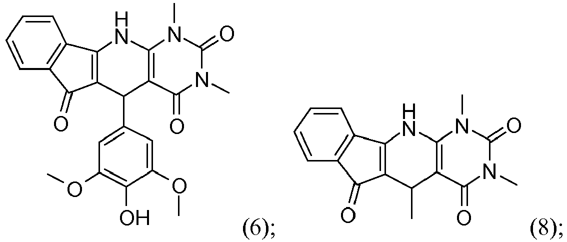 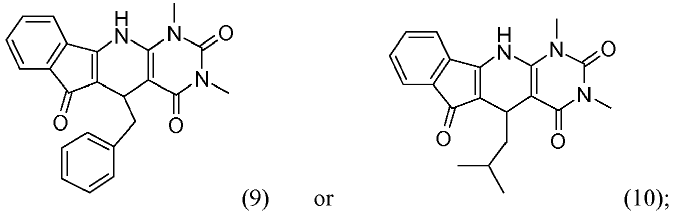 or a physiologically acceptable salt thereof.

According to one embodiment, compounds (1) to (3), (5) and (6), and preferably (3), (5) and (6), and even more preferentially compounds (3) and (6), or a physiologically acceptable salt thereof, may be considered more particularly for the invention.

According to another embodiment, compounds (6) and (8) to (10), or a physiologically acceptable salt thereof may also be preferred.

A physiologically acceptable salt of a compound of general formula (Ia) or (Ib) or (IIa) according to the invention may be a salt of a compound of general formula (Ia), (Ib) or (IIa) and of an alkali metal, of an alkaline-earth metal or of ammonium, comprising the salts obtained with organic ammonium bases, or salts of a compound of general formula (Ia), (Ib) or (IIa) and of an organic or mineral acid.

Salts that are more particularly suitable for use in the invention may be sodium, potassium, calcium or magnesium salts, quaternary ammonium salts such as tetramethylammonium or tetraethylammonium, and addition salts with ammonia and physiologically acceptable organic amines, such as methylamine, dimethylamine, trimethylamine, ethylamine, triethylamine, ethanolamine or tris(2-hydroxyethyl)amine.

Salts of a compound of general formula (Ia), (Ib) or (IIa) and of a mineral acid that are suitable for use in the invention may be obtained with hydrochloric acid, hydrobromic acid, sulfuric acid, nitric acid or phosphoric acid.

Salts of a compound of general formula (Ia), (Ib) or (IIa) and of an organic acid that are suitable for use in the invention may be obtained with carboxylic acids and sulfonic acids such as formic acid, acetic acid, oxalic acid, citric acid, lactic acid, malic acid, succinic acid, malonic acid, benzoic acid, maleic acid, fumaric acid, tartaric acid, methanesulfonic acid, benzenesulfonic acid or p-toluenesulfonic acid.

A compound of the invention may be used in a proportion of from 0.01% to 10% by weight and preferably from 0.1% to 5% by weight relative to the total weight of the composition containing it.

Compounds of the invention may be obtained according to any method known to those skilled in the art.

In particular, the pyrido-pyrimidine-trione derivatives of the invention may be obtained by means of the Hantzsch reaction allowing a one-step synthesis. The Hantzsch reaction is known in the literature (especially Biooog. Med. Chem. 2005, 13, 6678-6684), and is represented schematically below:

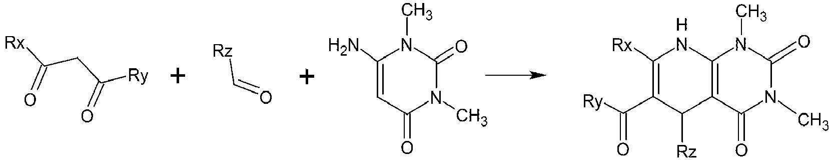

These compounds may be obtained using a Radley system, under an inert atmosphere, with condensing equipment and a hotplate, equipped with a magnetic stirrer. Various work-up and purification methods may be applied.

The Hantzsch reaction may occasionally lead to oxidized compounds: de-arylated product and/or dehydrogenated product (pyridine backbone). In certain cases, an additional reduction step using, for example, a reducing agent such as NaBH₄, may thus be performed.

### Compositions

A compound of the invention is advantageously formulated in a composition that may be in any galenical form normally available for the intended indication and mode of administration.

A composition according to the invention comprises a physiologically or pharmaceutically acceptable medium.

A composition according to the invention may be administered orally, topically, intradermally or subcutaneously, and preferably topically.

According to one embodiment, a topical composition according to the invention may advantageously be formulated in any galenical form that is suitable for caring for the skin and its integuments, and may be in the form of ointments, creams, solutions, gels, emulsions, foams or aerosol compositions containing a propellant, milks, pomades, powders, impregnated pads, lotions or suspensions. It may also be in the form of lipid or polymeric microspheres, nanospheres or vesicles or polymer patches and hydrogels allowing controlled release. These compositions may be in anhydrous or aqueous form depending on the intended dermocosmetic indication.

A composition intended for topical administration may be an aqueous, aqueous-alcoholic or oily solution, a solution or a dispersion of the lotion or serum type, an emulsion of liquid or semiliquid consistency of the milk type, obtained by dispersing a fatty phase in an aqueous phase (O/W) or conversely (W/O), a suspension or an emulsion, of soft, semisolid or solid consistency, of the cream type or of the aqueous or anhydrous gel type, a multiple emulsion (W/O/W or O/W/O), a microemulsion, a nanoemulsion, a preparation of microcapsules, a preparation of microparticles, a vesicular dispersion of ionic and/or nonionic type, or a wax/aqueous phase dispersion.

According to one embodiment, a composition of the invention may also be in the form of a transdermal system allowing active or passive release of the active agent(s) transdermally, for example of the patch or patch gel (hydrogel) type.

These compositions are prepared according to the usual methods.

In the case of a composition in accordance with the invention for oral administration, the use of an ingestible support, whose nature is adapted according to the type of composition under consideration, is preferred. Tablets, gel capsules or lozenges, suspensions, oral supplements in dry form and oral supplements in liquid form, milk, yoghurt, cheese, fermented milks, milk-based fermented products, ice creams, cereal-based products or fermented cereal-based products, milk-based powders, infant and baby formulae, food products of confectionery, chocolate or cereal type, and animal feed in particular for pets, are thus especially suitable as food supports.

The term "oral composition" means, for example, nutritional, nutraceutical, cosmeceutical or pharmaceutical compositions comprising at least one compound according to the invention.

The formulation of the oral compositions according to the invention may be performed via any common process known to those skilled in the art for producing drinkable solutions, coated tablets, gel capsules, gels, emulsions, tablets to be swallowed or chewed, capsules, especially soft or hard capsules, granules to be dissolved, syrups, solid or liquid foods and hydrogels allowing controlled release, food bars, powders, in compacted or non-compacted form, liquid solutions or suspensions, confectioneries, fermented milk, fermented cheeses, chewing gums, toothpastes or spray solutions.

The oral compositions may be either in anhydrous form or in aqueous form according to the dermocosmetic indication.

A compound of the invention may moreover be formulated with the usual excipients and components for such oral compositions or food supplements, i.e. especially fatty and/or aqueous components, humectants, thickeners, preserving agents, texture agents, taste agents and/or coating agents, and/or antioxidants.

The formulating agents and excipients for oral compositions, and especially for food supplements, are known in this field and will not be the subject of a detailed description herein.

According to another embodiment, a compound that is suitable for use in the invention may be used subcutaneously or intradermally.

In such a use, an active agent that is suitable for use in the invention may be conditioned in the form of an aqueous or nonaqueous sterile isotonic solution, in the form of a dispersion, suspension or emulsion prepared, where appropriate, just before administration, using a sterile powder, for example a freeze-dried powder, which is then reconstituted in the form of an injectable sterile solution or a dispersion at the time of use.

The term "sterile" is intended to qualify a formulation that is capable of ensuring the harmlessness required for intraepidermal and/or intradermal and/or subcutaneous administration.

A composition that is suitable for use in the invention may comprise any excipient usually used in the field of injectable sterile solutions.

The administration of a composition of the invention may be performed via any injection technique and/or device that is suitable for intraepidermal and/or intradermal and/or subcutaneous injection. Thus, such an administration may also be performed by mesotherapy.

Via the intradermal route, administration using a systemic patch may be preferred.

A composition according to the invention may also comprise any formulating agent or any cosmetically or dermatologically acceptable additional active agent. The amounts of these various active agents are those conventionally used in the field under consideration, and are especially determined so as not to affect the desired properties for a compound of the invention or for a composition of the invention.

A cosmetic or dermatological active agent that is suitable for use in the invention may be chosen from moisturizers, pro-desquamating agents, vitamins, essential fatty acids, hydroxy acids, polysaccharide sulfates, sphingolipids, UV-screening agents, antioxidants, antiacne agents, antiinflammatory agents, tanning agents (in the absence of UV radiation), depigmenting agents, matting agents, proteins or protein hydrolysates, probiotic agents, amino acids, polyols, urea and derivatives thereof, jasmonic acid and derivatives thereof, allantoin, sugars and sugar derivatives, water-soluble vitamins, plant extracts and hydroxy acids, retinol and derivatives thereof, tocopherol and derivatives thereof, essential fatty acids, ceramides, essential oils, salicylic acid and derivatives thereof, vitamin D3 and derivatives thereof, retinoids and derivatives thereof, PPAR-gamma agonists, hair-loss counteractants or hair regrowth agents such as potassium channel openers, antiperspirants, anti-seborrhoeic agents, antidandruff agents, antiandrogens, microbial enzymes which inhibit the maturation of lipids, in particular lipases, and mixtures of these active agents.

A composition of the invention may comprise any formulating agent usually used in the field. As examples of formulating agents that are suitable for use in the invention, mention may be made of hydrophilic gelling agents, lipophilic gelling agents, surfactants, fillers, odour absorbers, dyestuffs, film-forming polymers, fatty substances such as volatile or non-volatile oils, pasty or solid fatty substances, such as waxes or gums, solvents, such as ethanol or propylene glycol, or a spring water and/or mineral water.

### Conditioning article

A packaging article of the invention will naturally be chosen by a person skilled in the art according to the galenical form of the composition to be packaged.

Thus, for liquid compositions, use may be made of containers consisting of a rigid envelope comprising means for dispensing the composition. These distribution means may be a simple orifice, which is blocked by a removable cap, or a push button associated with a pump for expelling a part of the composition contained in the container. The compositions of the invention in liquid form may also be conditioned in containers of aerosol can type.

A composition of the invention in semiliquid or pasty form may be advantageously conditioned in a jar, a tube of cream, or in a container with flexible or deformable walls and having an orifice that can be stoppered with a removable lid, the composition being expelled through the orifice by pressing on the walls, or a bottle equipped with a push button and a pump as indicated previously.

According to a particular embodiment, a conditioning article that is suitable for receiving a composition according to the invention may be made of glass, metal, alloy, coated papers such as wax-coated papers, for instance papers coated with beeswax, which especially have properties as a natural preserving agent.

Alternatively, a composition according to the invention may be stored under vacuum, in a hermetically sealed airtight compartment, like, for example, a brick-carton under vacuum, commonly used in the food sector.

The conditioning article may be at least partly made of plastics or other suitable polymeric materials.

According to a particular embodiment, the conditioning article can also be made using materials which make it possible to isolate the composition from any light sources.

The packaging article can also be at least partly made of thermal insulation materials. By way of example of materials of this type, mention may be made of fabrics, fabrics made of glass fibres coated with silicone, textiles based on ceramic fibres, cellulose fibres, polystyrene, styrofoam, and packaging films. A cold gel or liquid may also be used as thermal insulation material.

According to a particular embodiment, the conditioning article may be disposable and opened just before its use by the consumer.

According to one embodiment, a composition of the invention formulated in solid form may be conditioned, for example, in a jar or a tube for a stick, for example a lipstick tube.

### Cosmetic treatment process

As indicated previously, the invention relates to a cosmetic process dedicated to individuals having, or liable to have, greasy skin or greasy-prone skin and/or an associated aesthetic skin disorder, in particular as defined above.

A process according to the invention may also comprise a step that consists in observing a reduction in, or even disappearance of, the aesthetic disorder under consideration.

Preferably, a process according to the invention may be performed topically, orally, intradermally, intraepidermally or subcutaneously.

Even more preferentially, a process according to the invention is performed topically or orally.

A process of the invention may be performed, topically, especially by applying to the skin or its integuments at least one coat of a cosmetic or dermatological composition comprising, as active agent, at least one compound of the invention, and more particularly a cosmetic composition as defined previously. The application may especially be performed on the skin of the face or the neckline, the scalp, the lips, the hair, the eyelashes or the nails, for example in the form of a mask.

A cosmetic process of the invention may be performed orally, especially by administration of at least one cosmetically-directed food composition comprising as active agent at least one compound of the invention, and in particular a cosmetically-directed food composition as defined above.

A cosmetic process of the invention may be performed on a daily basis for example, at a rate of, for example, a single administration per day or one administration twice a day, for example once in the morning and once in the evening.

A cosmetic process of the invention may be carried out over a time period ranging from one week to several weeks, or even several months, this period moreover possibly being repeated after periods without treatment, for several months or even several years.

By way of example, a compound of the invention may be administered, for example 2 to 3 times per day, or more, and generally over an extended period of at least 4 weeks, or even 4 to 15 weeks, with, where appropriate, one or more periods of interruption.

A process of the invention may advantageously comprise the administration of a composition of the invention, in combination, simultaneously, successively or separately over time, with the administration of an additional cosmetic or dermatological composition distinct from the composition of the invention and intended for caring for the scalp.

Any additional cosmetic or dermatological composition may be suitable for use in the invention, with the proviso, naturally, that its combination with a composition of the invention does not affect the properties desired for the same. A person skilled in the art knows how to assess, on the basis of his knowledge, the additional cosmetic or dermatological compositions that may be suitable for use.

In the description and the examples that follow, unless otherwise mentioned, the percentages are weight percentages and the ranges of values written in the form "between ... and ..." include the stated lower and upper limits.

The ingredients are mixed, before being formed, in the order and under conditions that may be readily determined by a person skilled in the art.

The content and nature of the ingredients used in the compositions of the invention are adjusted by a person skilled in the art so as not to substantially affect the properties required for the compositions of the invention.

### Figure legends

**Figure 1****:** illustrates an HES observation of an Episkin™ model on D13 after systemic treatment over 5 days with a compound of the invention.
**Figure 2****:** illustrates an immunolabelling of Ki67 performed using samples of Episkin™ treated in the absence and presence of a compound of the invention.
**Figure 3****:** illustrates western blotting of corneodesmosin performed using samples of Episkin™ treated in the absence and presence of a compound of the invention.

The examples below are given as non-limiting illustrations of the field of the invention.

### EXAMPLES

### Example 1

### Synthesis of compounds (1) to (6) and (8)

Compounds (1) to (6) and (8) of the invention may be obtained using a Radley system, under an inert atmosphere, with condensing equipment and a hotplate, equipped with a magnetic stirrer. Two work-up and purification methods may be applied.

The process detailed below concerns compound (2).

### Reagents

| **Reagents** | **Supplier** | **Purity** | **MW** | **n eq.** | **mmol** | **m (gr)** | **Volume** |
|---|---|---|---|---|---|---|---|
| **1**,**3**-**indanedione** | Aldrich 562606-368 | 97% | 146.15 | 1 | 34.21 | 1 | |
| **3-hydroxybenzaldehyde** | Acros A0275828 | 98.5% | 122.12 | 1 | 34.21 | 0.84 | |
| **6**-**amino**-**1**,**3-dimethyluracil** | Aldrich 569176-209 | 98% | 155.16 | 1 | 34.21 | 1.06 | |
| **Acetic acid** | Aldrich 61180 | | | 15V | | | 15 |

### Procedure:

Equipment: Radley tube equipped with a magnetic stirrer, placed under a nitrogen atmosphere. Indanedione (1 g), 3-hydroxybenzaldehyde (0.84 g) and 6-amino-1,3-dimethyluracil (1.06 g) are suspended in acetic acid (15 ml). The suspension is heated to reflux. Total dissolution of the reaction medium is observed, followed by the gradual formation of a yellow precipitate. The reaction progress is monitored by thin-layer chromatography (90/10 DCM/MeOH). After refluxing overnight, total disappearance of the indanedione is observed.

### Work-up and purification:

### Method A:

The heterogeneous reaction medium is filtered through a sinter funnel, and rinsed thoroughly with an ethanol/water mixture (1/1) and then with ethanol. The orange solid is then dried under vacuum in the presence of P₂O₅.

The solid is triturated with acetone (30 V) for 20 minutes, filtered through a sinter funnel and then dried under vacuum. 2.5 g of an orange solid are thus obtained. Yield: 94%.

### Method B:

For certain reaction media, the desired product does not precipitate out, even under cold conditions. The mixture is then concentrated to dryness by co-evaporation with toluene, and the residue is then purified by chromatography on silica gel (dichloromethane/methanol gradient), before being triturated in isopropyl ether. A final trituration in boiling water gives the desired compounds after filtration on a sinter funnel and drying over P₂O₅ under vacuum.

### Analytical description of the products:

### Compound (1)

Reagents used: 1,3-indanedione, 3-methoxybenzaldehyde, 6-amino-1,3-dimethyluracil. Purification according to method B.
Average yield: 44%; orange-red solid.
¹H NMR (300 MHz, DMSO-d6): coherent
LC/MS: x-Terra MS C18 4.6*150 mm column, 5 µm, HCOOH/MeCN, tr=10.69 min, > 98% at 256.3 nm; Mass ESI+: [M+H]⁺ = 402.0, [M+Na]⁺= 423.9; ESI-: [M-H]⁻= 400.9

### Compound (2)

Reagents used: 1,3-indanedione, 3-hydroxybenzaldehyde, 6-amino-1,3-dimethyluracil. Purification according to method A.
Average yield: 94%; orange solid.
¹H NMR (300 MHz, DMSO-d6): coherent
LC/MS: x-Terra MS C18 4.6*150 mm column, 5 µm, HCOOH/MeCN, tr=8.96 min, > 98% at 256.3 nm; Mass ESI+: [M+H]⁺= 388.1, [M+Na]⁺= 410.1, [MM+Na]⁺= 797.4; ESI-: [M-H]⁻ = 386.1

### Compounds (3)

Reagents used: 1,3-indanedione, 4-hydroxy-3-methoxybenzaldehyde, 6-amino-1,3-dimethyluracil. Purification according to method A.
Average yield: 41 %; orange solid.
¹H NMR (300 MHz, DMSO-d6): coherent
LC/MS: x-Terra MS C18 4.6*150 mm column, 5 µm, HCOOH/MeCN, tr=9.05 min, > 96% at 256.3 nm;
Mass ESI+: [M+Na]⁺ = 440.0; ESI-: [M-H]⁻ = 416.0

### Compound (4)

Reagents used: 1,3-indanedione, 4-methoxybenzaldehyde, 6-amino-1,3-dimethyluracil. Purification according to method A.
Average yield: 82%; orange solid.
¹H NMR (300 MHz, DMSO-d6): coherent
LC/MS: x-Terra M5 C18 4.6*150 mm column, 5 µm, HCOOH/MeCN, tr=10.59 min, > 99% at 256.3 nm; Mass ESI+: [M+Na]⁺ = 424.3; ESI-: [M-H]⁻= 400.3

### Compounds (5)

Reagents used: 1,3-indanedione, 4-hydroxybenzaldehyde, 6-amino-1,3-dimethyluracil. Purification according to method A.
Average yield: 56%; orange solid.
¹H NMR (300 MHz, DMSO-d6): coherent
LC/MS: x-Terra MS C18 4.6*150 mm column, 5 µm, HCOOH/MeCN, tr=8.9min, > 98% at 256.3 nm;
Mass ESI+: [M+Na]⁺= 410.2; ESI-: [M-H]⁻=386.2

### Compound (6)

Reagents used: 1,3-indanedione, 3,5-dimethoxy-4-hydroxybenzaldehyde, 6-amino-1,3-dimethyluracil. Purification according to method B.
Average yield: 45%; orange solid.
¹H NMR (300 MHz, DMSO-d6): coherent
LC/MS: x-Terra MS C18 4.6*150 mm column, 5 µm, HCOOH/MeCN, tr=8.89 min, > 96% at 257.5 nm; Mass ESI+: [M+Na]⁺ = 470.2; ESI-: [M-H]⁻ = 446.2

### Compound (8)

Reagents used: 1,3-indanedione, acetaldehyde, 6-amino-1,3-dimethyluracil. Purification according to method A.
Average yield: 52%; orange solid.
¹H NMR (300 MHz, DMSO-d6): coherent
LC/MS: no technique in coherence with the ¹H NMR spectrum (partial oxidation of the product); Mass ESI+: [M+Na]⁺ = 310.1; ESI-: [M-H]⁻= 308.1

### Example 2

### Synthesis of compounds (6), (9) and (10)

These compounds were obtained by using the protocol described previously (Example 1) completed by an additional reduction step. The reducing agent NaBH₄ allows the desired reduced product to be recovered.

The experimental procedure is detailed for compound (10).

### Reagents

| **Reagents** | **Supplier MW** | | n **eq.** | **mmol** | **m (gr)** | **Volume (ml)** |
|---|---|---|---|---|---|---|
| **Aromatic compound derived from the Hantzsch reaction** | | 349.3 | 1 | 6.30 | 2.2 | |
| **Sodium borohydride** | Acros A0201210001 | 37.83 | 1.5+1 | 15.75 | 0.36 + 0.24 | |
| **Tetrahydrofuran** | Aldrich | | 30V | | | 65 ml |

### Procedure:

Equipment: 100 ml three-necked flask equipped with a magnetic stirrer and a condenser, and placed under a nitrogen atmosphere.

The "aromatic" compound (2.2 g) is suspended in tetrahydrofuran (65 ml). Sodium borohydride (360 mg) is added portionwise at room temperature, and the mixture is then gradually heated to 45°C. Monitoring of the progress by thin-layer chromatography (TLC), after 3 hours of reaction, shows the presence of starting material. Further sodium borohydride (240 mg) is added portionwise to the reaction medium. After stirring for 3 hours at 45°C, traces of starting material are visible by TLC (90/10 DCM/MeOH).

### Work-up and purification:

The reaction medium is poured into an ice/water mixture. The precipitate observed is filtered off and rinsed with water. After drying under vacuum, the residue is triturated in isopropyl ether, filtered off on a sinter funnel and then triturated again in boiling water. After drying under vacuum in the presence of P₂O₅, a white solid is obtained: 1.9 g (yield: 86%).

### Analytical description of the products:

### Compounds (6)

Average yield: 63%; white solid.
¹H NMR (300 MHz, DMSO-d6): coherent
LC/MS: x-Terra MS C18 4.6*150 mm column, 5 µm, HCOOH/MeCN, tr=11.79 min, > 96% at 210.0 nm; Mass ESI+: [M+H]⁺ =372.2; ESI-: [M-H]⁻= 370.1

### Compound (7)

Average yield: 38%; white solid.
¹H NMR (300 MHz, DMSO-d6): coherent
LC/MS: x-Terra MS C18 4.6*150 mm column, 5 µm, HCOOH/MeCN, tr=12.48 min, > 94% at 210.0 nm; Mass ESI+: [M+H]⁺=386.1; ESI-: [M-H]⁻= 384.0

### Compound (8)

Average yield: 86%; white solid.
¹H NMR (300 MHz, DMSO-d6): coherent
LC/MS: x-Terra MS C18 4.6*150 mm column, 5 µm, HCOOH/MeCN, tr=12.62 min, > 96% at 210.0 nm; Mass ESI+: [M+H]⁺=352.2; ESI-: [M-H]⁻= 350.2

### Example 3

### Characterization of the compounds of the invention on a model of in vitro skin

The study proceeded in three steps:
- The molecules first modelled and synthesized were used to treat Episkin™ reconstructed epidermis kits.
- Next, a protein study on histological slices and on cell extracts was performed.
- In a second stage, a selection of molecules was evaluated on a RealSkin™ model.

### Materials and methods

### Episkin™ reconstructed epidermis,

The samples of reconstructed epidermis, received from the company Episkin at six days of reconstruction, were recultured as soon as received in 3.5 ml of differentiation medium (Episkin™ medium) under insert (in immersion) for 48 hours in an incubator (37°C, 5% CO₂).

The epidermis samples were then placed in contact with the test compounds systemically. The Episkin™ differentiation culture medium is changed for a differentiation medium containing the test compounds with 0.1% of final DMSO vehicle. The epidermis samples were recultured in an incubator for 48 hours. A new systemic treatment is performed for a further 72 hours of culture in the incubator.

For the treatment on Episkin™, each test compound was prepared extemporaneously by weighing out and dissolving at 10 mM in DMSO, and was then used to treat 3 samples of reconstructed epidermis. Furthermore, each test compound was used in a proportion of 1 and 10 µM final in the culture medium (0.1% DMSO final). The positive controls used were vitamin D3, retinol and calcipotriol (a vitamin D3 analogue).

The epidermis samples were collected (3 epidermis samples per treatment) for histological analysis and protein extracts (the same reconstructed epidermis is used for the histological study and the protein extract).

### Histological preparations

The morphological control of the treated reconstructed epidermis samples was performed by staining with HES (haematoxylin-eosin-saffron) according to a standard protocol on histological slices of the samples included in paraffin (toluene bath: three times 15 minutes; 100° alcohol baths: twice for 1 minute; 95° alcohol baths: 1 minute; rinsing in water baths; haematoxylin bath: once for 2 minutes; rinsing in water baths; short dipping in a hydrochloric acid bath; rinsing in water; eosin bath: once for 1 minute; rinsing in water; short dipping in a 100° alcohol bath; saffron bath (5 minutes); short dipping in a 100° alcohol bath; toluene bath: three times for 2 minutes).

### Fluorescent staining

The epidermis samples were included in OCT for freezing in liquid nitrogen and then stored at -80°C. The blocks obtained were sliced using a cryostat to a thickness of 7 µm, and the slices were dried and stored at -20°C while awaiting immunolabelling. For the chosen markers, the antibodies used and the dilutions thereof are listed in the following table:

| | | |
|---|---|---|
| anti-mouse Ki67 antibody | Novocastra MM1 | Dilution 1/20 |

### Western blots

The remaining epidermis from each sample was milled in a vibromill (Retsch MM400) 2x30 Hz, 3 minutes; with a stainless-steel ball 3 mm in diameter (Retsch No. 22.455.0002) in 300 µl of Natif+ buffer: Tris buffer (thermo No. 28376); 1M NaCl final (Sigma No. 57653); Triton X100 1% final (Calbiochem No. 648466); protease inhibitors (complete Roche No. 11873580), pepstatin 1 µM final (Roche No. 1359053).

The extract was then centrifuged at 14 000 rpm for 5 minutes and the supernatant was filtered through a 0.45 µm Ultrafree membrane (Millipore UFC30HVNB). The extracts were assayed by the BCA method (Pierce No. 23225) according to the supplier's protocol and equilibrated at 0.3 mg/ml.

| | | |
|---|---|---|
| Mouse antibody anti-corneodesmosin | Abnova 1041-A01 | Dilution 1/3000 |

### Results

### Morphological observations

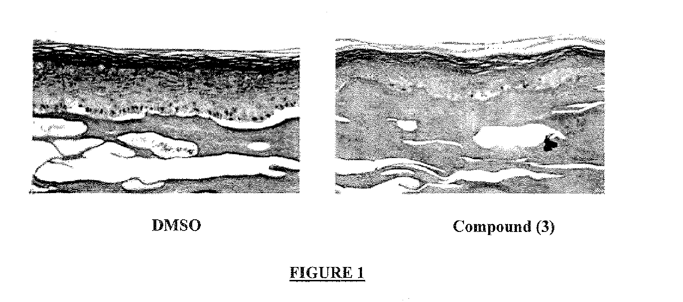

Figure 1 illustrates an HES observation of an Episkin model on D13 after systemic treatment over 5 days with compound (3).

The effect observed on the general structure of the epidermis, illustrated by Figure 1, shows that the compounds of the invention induce and stimulate a more or less accentuated disappearance of the live layers pushed towards the accelerated formation of a more or less thickened *stratum corneum*.

### Results of the immunolabellings

The marker chosen for the immunolabellings is Ki67, which affords information regarding a possible modification of the cell proliferation in the basal layer.

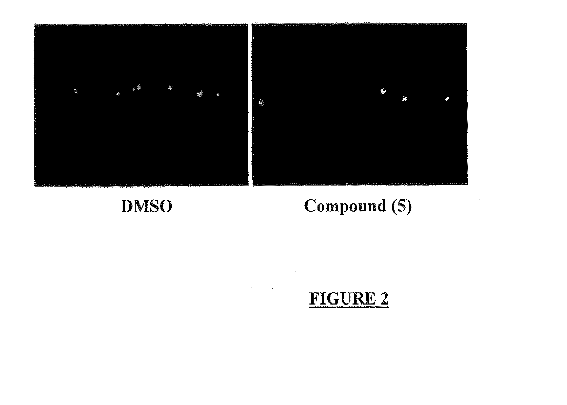

The results, illustrated by Figure 2 with compound (5) of the invention, show that the compounds of the invention induce a marked, or even occasionally total, decrease of the Ki67 labelling, and therefore have strong antiproliferative properties.

### Proteomic results

The marker chosen in proteomics is corneodesmosin, which affords information regarding a possible activation of proteolytic maturation of the *stratum corneum*. In general, in the reconstructed skin models, this maturation is greatly limited.

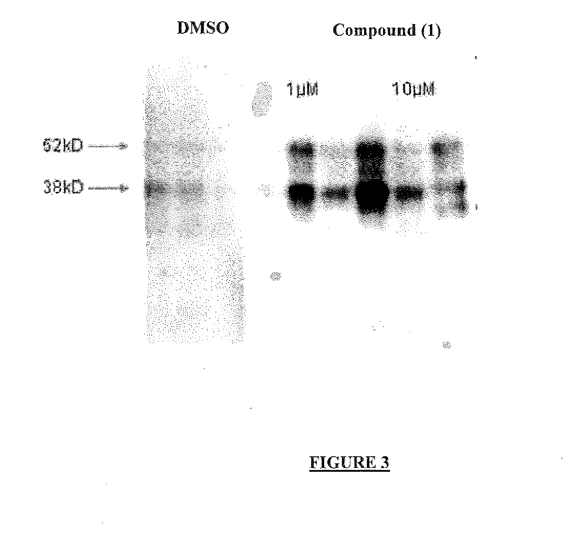

The results, illustrated by Figure 3 with compound (1) of the invention, show that the compounds of the invention induce an increase of the soluble fragments of corneodesmosin, reflecting an acceleration of the phenomenon of maturation of the *stratum corneum* and/or an increase in the number of SC layers.

### Summary table of the results

The results detailed above are collated in the table below. For each of the parameters monitored, the grade for the compound that is the most active among the test compounds (effect greater or lesser than the reference) is indicated by an asterisk.

The effect is generally graded for the highest concentration used (10 µM). Certain compounds showed effects at the lowest test concentrations (1 µM). The relative classification in this table takes this into account.

| **Compound** | HES | CDSN | Ki67 |
|---|---|---|---|
| Vitamin D3 | 0 | 0 | 0/- |
| Retinol (100 µM) | 0 | 0/+ | 0 |
| Calcipotriol | ++ | + | -- |
| (1) | + | ++(*) | - |
| (2) | + | ++ | 0 |
| (3) | ++ | + | - |
| (5) | + | + | - |
| (6) | ++(*) | + | - |

| | | | |
|---|---|---|---|
| **Abbreviations in the table: HES** = Haematoxylin - Eosin - Saffron (H.E.S.) **CDSN** = Corneodesmosin | | | |

The above results reveal that the compounds of the invention have properties of vitamin D3 type, which are particularly advantageous, for preventing and/or treating greasy skin or greasy-prone skin and/or an associated aesthetic skin disorder.

### Example 4

| *Formulation of the invention* | |
|---|---|
| - Compound (6) | 2% by weight of Active Material (AM) |
| - Sodium acrylamido-2-methylpropanesulfonate/ | |
| 2-hydroxyethyl methacrylate copolymer at 90% in water | |
| (Sepinov EMT 10 from SEPPIC) | 1.8% AM, i.e. 2% per se |
| - Capric/caprylic acid triglycerides | |
| (Miglyol 812 N by the company Sasol) | 5% |
| - water | qs 100% |

## Claims

1. Cosmetic use, as an agent for preventing and/or treating greasy skin or greasy-prone skin and/or an associated aesthetic skin disorder, of an effective amount of at least one compound represented by either of the general formulae (Ia) and (Ib): 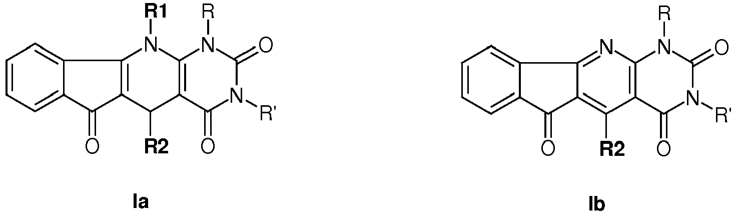 in which:
- R¹ represents H or a group -C(O)R⁸, with R⁸ being a linear or branched, saturated or unsaturated C₁-C₄ alkyl, or being a phenyl;
- R² represents H; a linear or branched, saturated or unsaturated C₁-C₁₂ alkyl group; or a group chosen from: 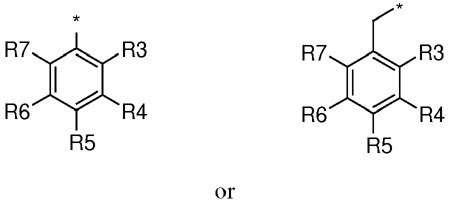 in which R³, R⁴, R⁵, R⁶ and R⁷ represent, independently of each other, H; -NO₂ -OH; -OR⁸; -CN; Br; Cl; I; F; -CF₃; a linear or branched, saturated or unsaturated C₁-C₄ alkyl; a phenyl; -OC(O)-R⁸; -O-Ph-X with X representing H, -OH, -NO₂, a fluorine, a linear or branched, saturated or unsaturated C₁-C₄ alkyl or alkoxy; R⁸ being as defined previously,
- R and R', which may be identical or different, represent H or a linear or branched C₁-C₄ alkyl,
or a physiologically acceptable salt thereof.

2. Use according to Claim 1, in which the said compound is of general formula (Ia) in which:
- R¹ represents H,
- R² represents H; a linear or branched, saturated or unsaturated C₁-C₁₂ alkyl group; or a group chosen from: 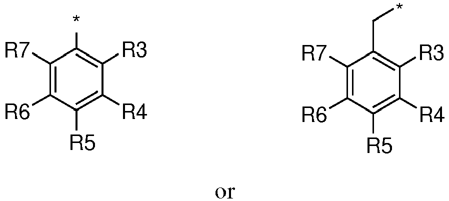 in which R³, R⁴, R⁵, R⁶ and R⁷ represent, independently of each other, H; -NO₂ -OH; -OR⁸; -CN; F; -CF₃; a linear or branched, saturated or unsaturated C₁-C₄ alkyl; a phenyl; -OC(O)-R⁸; -O-Ph-X with X representing H, -OH, -NO₂, a fluorine, a linear or branched, saturated or unsaturated C₁-C₄ alkyl or alkoxy; with R⁸ being a linear or branched, saturated or unsaturated C₁-C₄ alkyl, or being a phenyl,
- R and R', which may be identical or different, represent H or a linear or branched C₁-C₄ alkyl,
or a physiologically acceptable salt thereof.

3. Use according to Claim 1 or 2, in which the said compound is of general formula (Ia) in which:
- R¹ represents H,
- R² represents H; a linear or branched, saturated or unsaturated C₁-C₁₂ alkyl group; or a group chosen from: 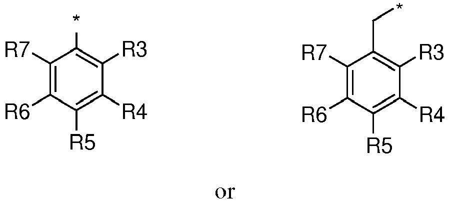 in which R³, R⁴, R⁵, R⁶ and R⁷ represent, independently of each other, H; -OH; -OR⁸; -CN; a linear or branched, saturated or unsaturated C₁-C₄ alkyl; a phenyl; -OC(O)-R⁸; - O-Ph-X with X representing H, -OH, a linear or branched, saturated or unsaturated C₁-C₄ alkyl or alkoxy; with R⁸ being a linear or branched, saturated or unsaturated C₁-C₄ alkyl, or being a phenyl,
- R and R', which may be identical or different, represent H or a methyl,
and physiologically acceptable salts thereof.

4. Use according to one of the preceding claims, in which the said compound is of general formula (Ia) in which:
- R¹ represents H,
- R² represents H, a linear or branched, saturated C₁-C₆ alkyl group; or a group chosen from: 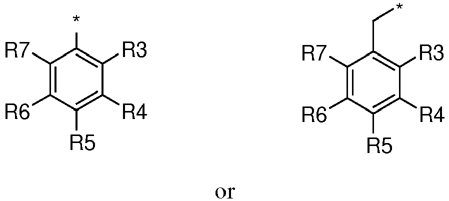 in which R³, R⁴, R⁵, R⁶ and R⁷ represent, independently of each other, H; -OH; -OR⁸; -CN; a linear or branched, saturated or unsaturated C₁-C₄ alkyl; R⁸ being a linear or branched, saturated or unsaturated C₁-C₄ alkyl, or being a phenyl,
- R and R' represent a methyl,
or a physiologically acceptable salt thereof.

5. Use according to one of the preceding claims, in which the said compound is chosen from: 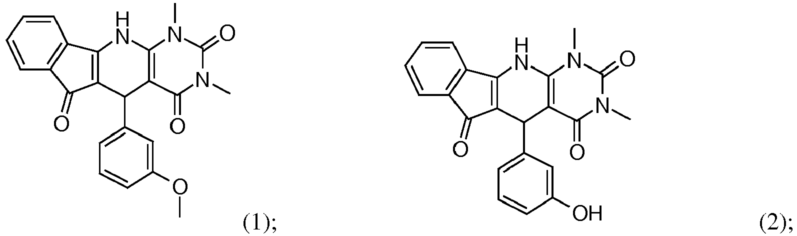 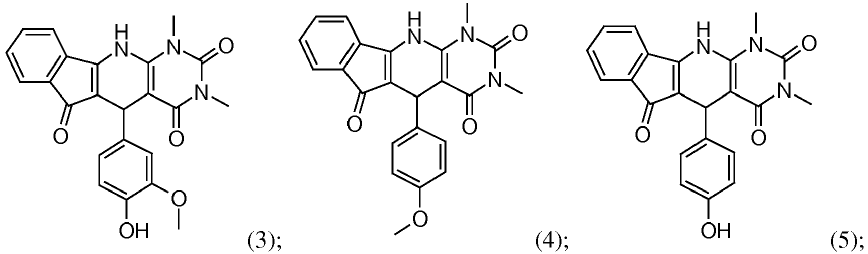 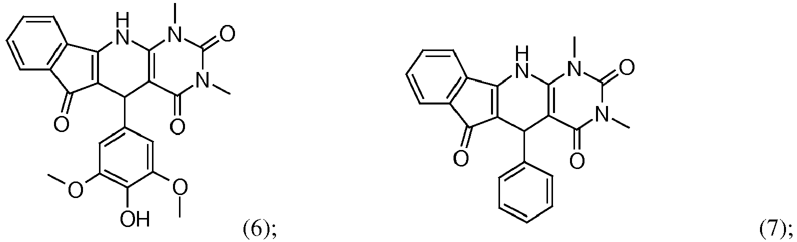 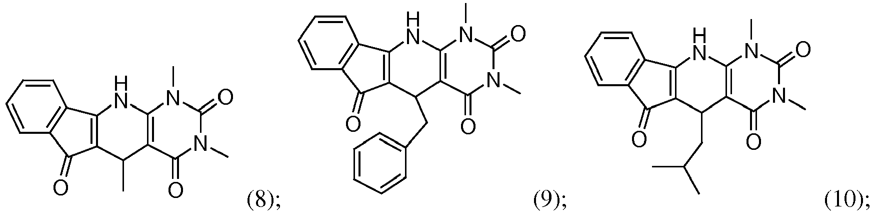 or 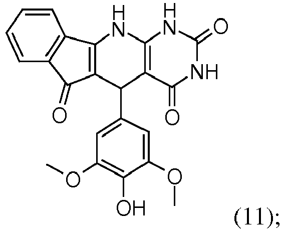 or a physiologically acceptable salt.

6. Use according to one of the preceding claims, in which the said compound is used in a proportion of from 0.01% to 10% by weight and preferably from 0.1% to 5% by weight relative to the total weight of the composition containing it.

7. Use according to one of the preceding claims, in which the aesthetic disorder is chosen from skin imperfections caused by hyperseborrhoea, and/or a scalp disorder.

8. Use according to one of the preceding claims, in which the aesthetic disorder is chosen from a thick skin grain, shiny skin, skin with dilated pores or follicular orifices, skin with pores or follicular orifices filled with cornified spicules or with comedones, dyschromia, redness, rough skin with, where appropriate, dry skin plaques, or skin with an impaired complexion.

9. Use according to the preceding claim, in which the skin complexion impairment is chosen from a sallow, non-uniform, dull, waxy or sickly complexion.

10. Compound as defined according to one of Claims 1 to 6, in an effective amount in a pharmaceutical or dermatological composition, for preventing and/or treating a pathological disorder of greasy skin or greasy-prone skin chosen from seborrhoeic dermatitis, acne and seborrhoeic pruritus.

11. Cosmetic process for treating and/or preventing greasy skin or greasy-prone skin and/or an associated aesthetic skin disorder, in an individual in need thereof, comprising at least one step of administering, to the said individual, at least an effective amount of at least one compound as defined according to one of Claims 1 to 5.

12. Compound represented by the general formula (IIa): 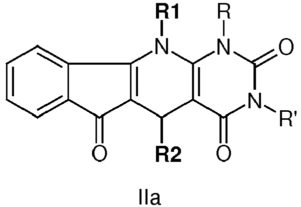 in which:
- R¹ represents H, and
- R² represents a linear or branched, saturated C₁-C₄ alkyl; a benzyl; or 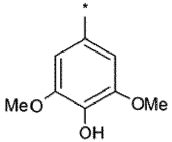
- R and R' represent a methyl, or a physiologically acceptable salt thereof.

13. Cosmetic or dermatological composition comprising, in a physiologically acceptable medium, an efficient amount of at least one compound represented by the general formula (IIa) as defined in claim 12.

## Patentansprüche

1. Kosmetische Verwendung, als Mittel zur Verhinderung und/oder Behandlung von fettiger oder tendenziell fettiger Haut und/oder damit einhergehendem Haut-Schönheitsfehler, einer wirksamen Menge von mindestens einer Verbindung, die durch eine der allgemeinen 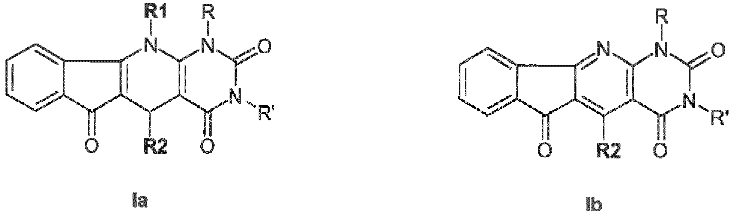 Formeln (Ia) oder (Ib) dargestellt ist:
wobei:
- R¹ H; oder eine Gruppe -C(O)R⁸ darstellt, wobei R⁸ ein lineares oder verzweigtes gesättigtes oder ungesättigtes C₁-C₄-Alkyl ist, oder es ist ein Phenyl;
- R² H; eine lineare oder verzweigte gesättigte oder ungesättigte C₁-C₁₂-Alkylgruppe oder eine Gruppe darstellt, die ausgewählt ist aus: 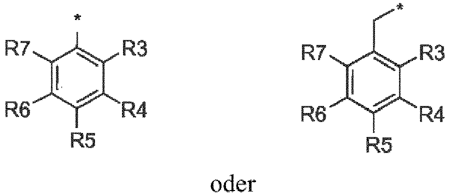 wobei R³, R⁴, R⁵, R⁶ und R⁷ unabhängig voneinander H; -NO₂; -OH, OR⁸; -CN, Br; Cl; I; F; -CF₃, ein lineares oder verzweigtes gesättigtes oder ungesättigtes C₁-C₄-Alkyl, ein Phenyl; -OC(O)-R⁸; -O-Ph-X darstellen, wobei X H, -OH, -NO₂, ein Fluor, ein lineares oder verzweigtes gesättigtes oder ungesättigtes C₁-C₄-Alkyl oder -Alkoxy darstellt, wobei R⁸ wie zuvor definiert ist,
- R und R', die gleich oder verschieden sein können, H oder ein lineares oder verzweigtes C₁-C₄-Alkyl darstellen,
oder eines physiologisch verträglichen Salzes davon.

2. Verwendung nach Anspruch 1, wobei die Verbindung die allgemeine Formel (Ia) aufweist, wobei:
- R¹ H darstellt,
- R² H, eine lineare oder verzweigte gesättigte oder ungesättigte C₁-C₁₂-Alkylgruppe, oder eine Gruppe ausgewählt aus: 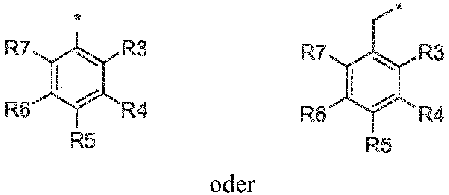 darstellt, wobei R³, R⁴, R⁵, R⁶ und R⁷ unabhängig voneinander H; -NO₂; -OH; OR⁸; -CN; F, -CF₃; ein lineares oder verzweigtes gesättigtes oder ungesättigtes C₁-C₄-Alkyl; ein Phenyl; -OC(O)-R⁸; -O-Ph-X darstellen, wobei X H, -OH, -NO₂, ein Fluor, ein lineares oder verzweigtes gesättigtes oder ungesättigtes C₁-C₄-Alkyl oder -Alkoxy darstellt, wobei R⁸ ein lineares oder verzweigtes gesättigtes oder ungesättigtes C₁-C₄-Alkyl darstellt, oder ein Phenyl ist,
- R und R', die gleich oder verschieden sein können, H oder ein lineares oder verzweigtes C₁-C₄-Alkyl darstellen,
oder ein physiologisch verträgliches Salz davon.

3. Verwendung nach Anspruch 1 oder 2, wobei die Verbindung die allgemeine Formel (Ia) aufweist, wobei:
- R¹ H darstellt;
- R² H; eine lineare oder verzweigte gesättigte oder ungesättigte C₁-C₁₂-Alkylgruppe oder eine Gruppe darstellt, die ausgewählt ist aus: 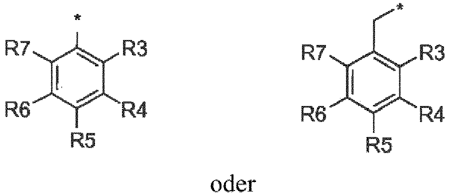 wobei R³, R⁴, R⁵, R⁶ und R⁷ unabhängig voneinander H; -OH, OR⁸; -CN, ein lineares oder verzweigtes gesättigtes oder ungesättigtes C₁-C₄-Alkyl, ein Phenyl -OC(O)-R⁸; -O-Ph-X darstellen, wobei X H, -OH, ein lineares oder verzweigtes gesättigtes oder ungesättigtes C₁-C₄-Alkyl oder -Alkoxy darstellt, wobei R⁸ ein lineares oder verzweigtes gesättigtes oder ungesättigtes C₁-C₄-Alkyl ist, oder es ist ein Phenyl;
- R und R', die gleich oder verschieden sind, H oder ein Methyl darstellen,
und physiologisch verträgliche Salze davon.

4. Verwendung nach einem der vorangehenden Ansprüche, wobei die Verbindung die allgemeine Formel (Ia) aufweist, wobei:
- R¹ H darstellt,
- R² H, eine lineare oder verzweigte gesättigte oder ungesättigte C₁-C₆-Alkylgruppe oder eine Grupp darstellt, die ausgewählt ist aus: 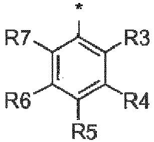 oder 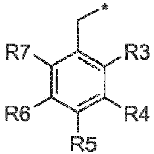 wobei R³, R⁴, R⁵, R⁶ und R⁷ unabhängig voneinander H; -NO₂; -OH; OR⁸; -CN; ein lineares oder verzweigtes gesättigtes oder ungesättigtes C₁-C₄-Alkyl darstellen, wobei R⁸ ein lineares oder verzweigtes gesättigtes oder ungesättigtes C₁-C₄-Alkyl darstellt, oder ein Phenyl ist,
- R und R' ein Methyl darstellen,
oder ein physiologisch verträgliches Salz davon.

5. Verwendung nach einem der vorangehenden Ansprüche, wobei die Verbindung ausgewählt ist aus: 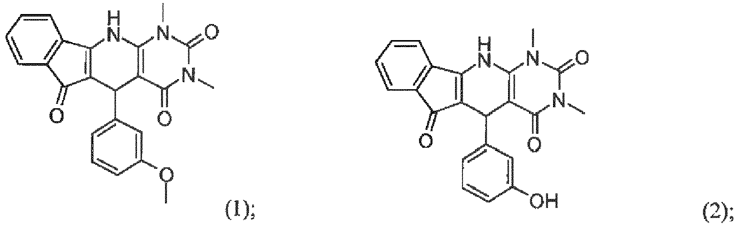 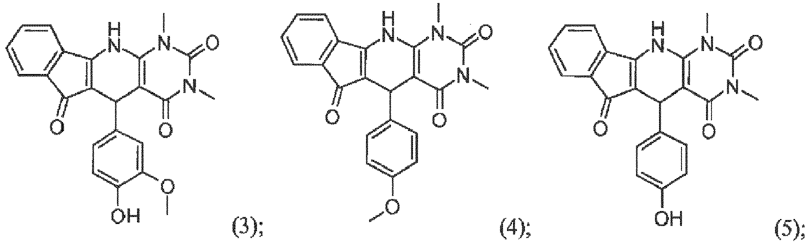 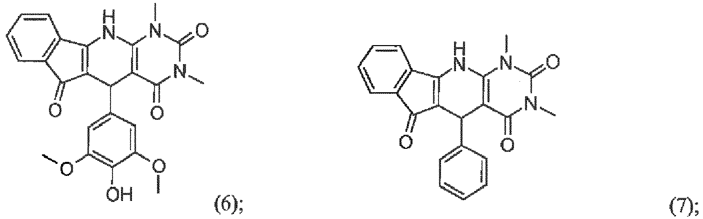 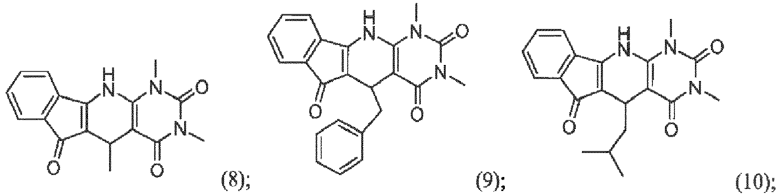 oder 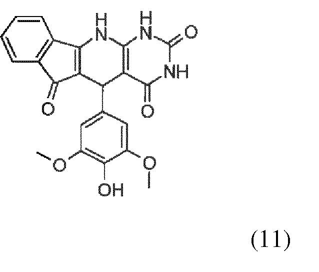 oder einem physiologisch verträglichen Salz.

6. Verwendung nach einem der vorangehenden Ansprüche, wobei die Verbindung in einem Anteil von 0,01 bis 10 Gew.-%, vorzugsweise von 0,1 bis 5 Gew.-%, relativ zu dem Gesamtgewicht der Zusammensetzung, die sie enthält, verwendet wird.

7. Verwendung nach einem der vorangehenden Ansprüche, wobei der Schönheitsfehler ausgewählt ist aus durch Hyperseborrhö und/oder einer Kopfhaut-Störung ausgelösten Hautunzulänglichkeiten.

8. Verwendung nach einem der vorangehenden Ansprüche, wobei der Schönheitsfehler ausgewählt ist aus einem dicken Hautkorn, glänzender Haut, Haut mit erweiterten Poren oder erweiterten Follikelöffnungen, Haut mit Poren oder Follikel-Öffnungen, die mit verhornten Nädelchen oder mit Komedonen gefüllt sind, Dyschromie, Rötung, rauher Haut mit, sofern zutreffend, trockenen Hautstellen oder Haut mit einem beeinträchtigten Hautbild.

9. Verwendung nach einem der vorangehenden Ansprüche, wobei die Hautbild-Beeinträchtigung ausgewählt ist aus einem fahlen, nicht gleichmäßigen, wachsartigen oder kränklichen Hautbild.

10. Verbindung nach einem der Ansprüche 1 bis 6 in einer wirksamen Menge in einer pharmazeutischen oder dermatologischen Zusammensetzung zur Verhinderung und/oder Behandlung einer pathologischen Störung von fettiger Haut oder tendentiell fettiger Haut, ausgewählt aus seborrhoischer Dermatitis, Akne und seborrhoischem Pruritus.

11. Kosmetisches Verfahren zur Behandlung und/oder Verhinderung von fettiger Haut tendentiell fettiger Haut und/oder einem damit zusammenhängenden Haut-Schönheitsfehler in einem Individuum mit Bedarf daran, umfassend mindestens einen Schritt der Verabreichung, mindestens einer wirksamen Menge von mindestens einer Verbindung nach einem der Ansprüche 1 bis 5 an das Individuum.

12. Verbindung, dargestellt durch die allgemeine Formel (IIa): 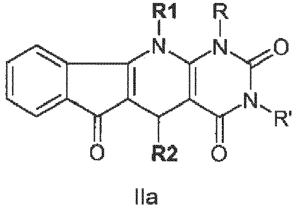 wobei:
- R¹ H darstellt, und
- R² ein lineares oder verzweigtes gesättigtes C₁-C₄-Alkyl, ein Benzyl oder 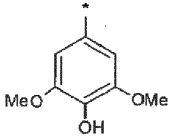 darstellt,
- R und R' ein Methyl darstellen, oder ein physiologisch verträgliches Salz davon.

13. Kosmetische oder dermatologische Zusammensetzung, umfassend, in einem physiologisch verträglichen Medium, eine wirksame Menge von mindestens einer Verbindung dargestellt durch die allgemeine Formel (IIa) wie in Anspruch 12 definiert.

## Revendications

1. Utilisation cosmétique, comme agent pour prévenir et/ou traiter une peau grasse ou à tendance grasse et/ou un désordre esthétique cutané associé, d'une quantité efficace d'au moins un composé représenté par une des formules générales (Ia) ou (Ib) : 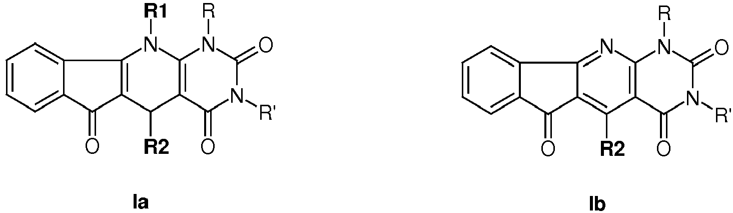 dans lesquelles :
- R¹ représente H ou un groupe -C(O)R⁸, R⁸ étant un groupe alkyle en C₁-C₄, linéaire ou ramifié, saturé ou insaturé, ou étant un groupe phényle ;
- R² représente H ; un groupe alkyle en C₁-C₁₂, linéaire ou ramifié, saturé ou insaturé ; ou un groupe choisi parmi : 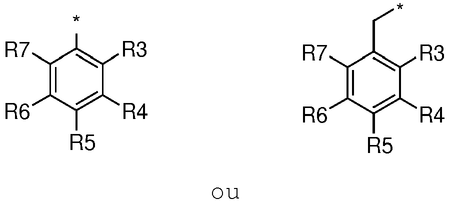 dans lesquels R³, R⁴, R⁵, R⁶, R⁷ représentent, indépendamment les uns des autres, H ; -NO₂ ; -OH ; -OR⁸ ; -CN ; Br ; Cl ; I ; F ; -CF₃ ; un groupe alkyle en C₁-C₄, linéaire ou ramifié, saturé ou insaturé ; un groupe phényle ; -OC(O)-R⁸ ; -O-Ph-X, X représentant H, -OH, -NO₂, un atome de fluor, un groupe alkyle ou alcoxy en C₁-C₄, linéaire ou ramifié, saturé ou insaturé ; R⁸ étant tel que défini précédemment,
- R et R', qui peuvent être identiques ou différents, représentent H ou un groupe alkyle en C₁-C₄, linéaire ou ramifié,
ou un sel physiologiquement acceptable de celui-ci.

2. Utilisation selon la revendication 1, dans laquelle ledit composé est de formule générale (Ia) dans laquelle :
- R¹ représente H,
- R² représente H ; un groupe alkyle en C₁-C₁₂, linéaire ou ramifié, saturé ou insaturé ; ou un groupe choisi parmi : 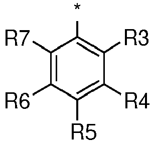 ou 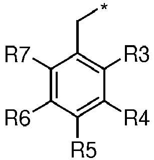 dans lesquels R³, R⁴, R⁵, R⁶, R⁷ représentent, indépendamment les uns des autres, H ; -NO₂ ; -OH ; - OR⁸ ; -CN ; F ; -CF₃ ; un groupe alkyle en C₁-C₄, linéaire ou ramifié, saturé ou insaturé ; un groupe phényle ; -OC(O)-R⁸ ; -O-Ph-X, X représentant H, -OH, -NO₂, un atome de fluor, un groupe alkyle ou alcoxy en C₁-C₄, linéaire ou ramifié, saturé ou insaturé ; R⁸ étant un groupe alkyle en C₁-C₄, linéaire ou ramifié, saturé ou insaturé, ou étant un groupe phényle,
- R et R', qui peuvent être identiques ou différents, représentent H ou un groupe alkyle en C₁-C₄, linéaire ou ramifié,
ou un sel physiologiquement acceptable de celui-ci.

3. Utilisation selon la revendication 1 ou 2, dans laquelle ledit composé est de formule générale (Ia) dans laquelle :
- R¹ représente H,
- R² représente H ; un groupe alkyle en C₁-C₁₂, linéaire ou ramifié, saturé ou insaturé ; ou un groupe choisi parmi : 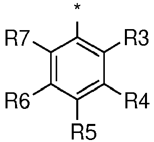 ou 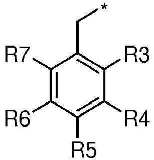 dans lesquels R³, R⁴, R⁵, R⁶, R⁷ représentent, indépendamment les uns des autres, H ; -OH ; -OR⁸ ; - CN ; un groupe alkyle en C₁-C₄, linéaire ou ramifié, saturé ou insaturé ; un groupe phényle ; -OC(O)-R⁸ ; - O-Ph-X, X représentant H, -OH, un alkyle ou un alkoxy en C₁-C₄, linéaire ou ramifié, saturé ou insaturé ; R⁸ étant un groupe alkyle ou alcoxy en C₁-C₄, linéaire ou ramifié, saturé ou insaturé ; R⁸ étant un groupe alkyle en C₁-C₄, linéaire ou ramifié, saturé ou insaturé, ou étant un groupe phényle,
- R et R', qui peuvent être identiques ou différents, représentent H ou un groupe méthyle,
et leurs sels physiologiquement acceptables.

4. Utilisation selon l'une des revendications précédentes, dans laquelle ledit composé est de formule générale (Ia) dans laquelle :
- R¹ représente H,
- R² représente H, un groupe alkyle en C₁-C₆, linéaire ou ramifié, saturé ; ou un groupe choisi parmi : 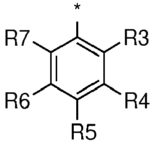 ou 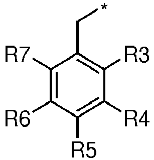 dans lesquelles R³, R⁴, R⁵, R⁶, R⁷ représentent, indépendamment les uns des autres, H ; -OH ; -OR⁸; -CN ; un groupe alkyle en C₁-C₄, linéaire ou ramifié, saturé ou insaturé ; R⁸ étant un groupe alkyle en C₁-C₄, linéaire ou ramifié, saturé ou insaturé, ou étant un groupe phényle,
- R et R' représentent un groupe méthyle,
ou un sel physiologiquement acceptable de celui-ci.

5. Utilisation selon l'une des revendications précédentes, dans laquelle ledit composé est choisi parmi : 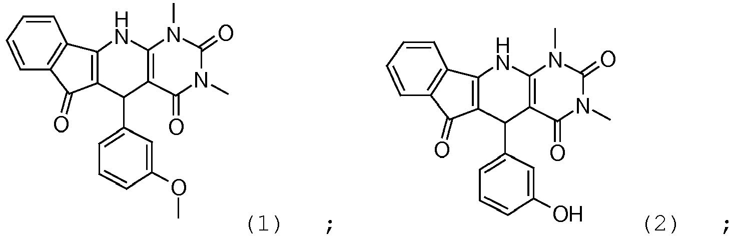 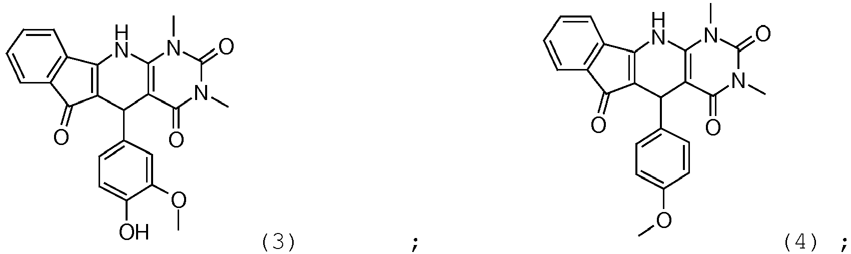 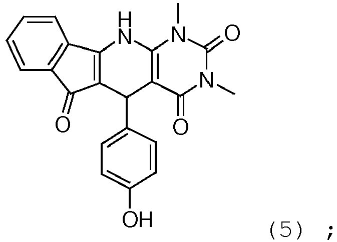 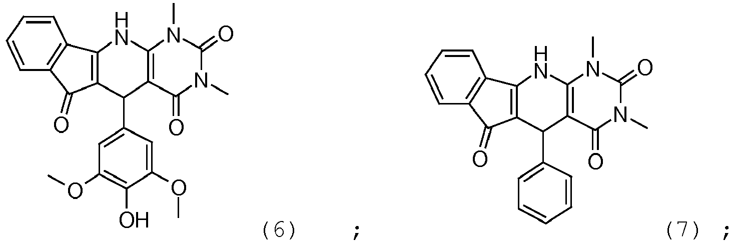 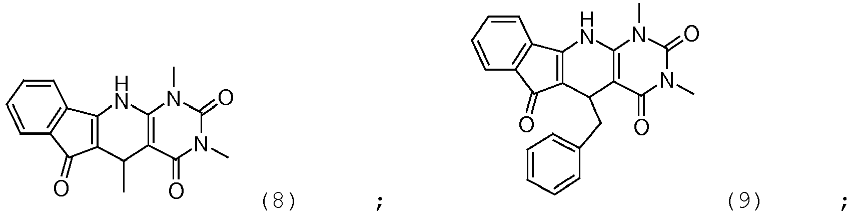 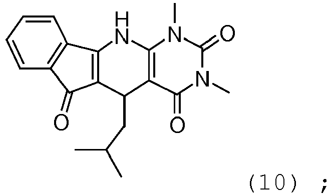 ou 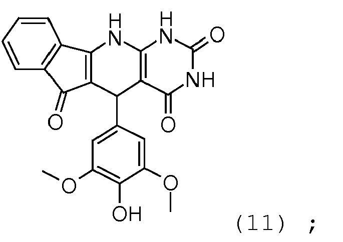 ou un sel physiologiquement acceptable.

6. Utilisation selon l'une des revendications précédentes, dans laquelle ledit composé est utilisé à une concentration de 0,01 à 10% en poids, de préférence de 0,1 à 5% en poids par rapport au poids total de la composition le contenant.

7. Utilisation selon l'une des revendications précédentes, dans laquelle le désordre esthétique est choisi parmi des imperfections cutanées dues à une hyperséborrhée, et/ou un désordre du cuir chevelu.

8. Utilisation selon l'une des revendications précédentes, dans laquelle le désordre esthétique est choisi parmi un grain de peau épais, une peau luisante, une peau présentant des orifices folliculaires ou des pores dilatés, une peau présentant des orifices folliculaires ou des pores remplis de spicules cornés ou de comédons, une dyschromie, une rougeur, une peau rugueuse avec, le cas échéant, des plaques de peau sèche, ou une peau présentant une altération du teint.

9. Utilisation selon la revendication précédente, dans laquelle l'altération du teint de la peau est choisie parmi un teint brouillé, inhomogène, terne, cireux ou maladif.

10. Composé tel que défini selon l'une des revendications 1 à 6, en quantité efficace dans une composition pharmaceutique ou dermatologique, pour prévenir et/ou traiter un désordre pathologique de la peau grasse ou à tendance grasse choisi parmi la dermatite séborrhéique, l'acné, et le prurit séborrhéique.

11. Procédé cosmétique pour traiter et/ou prévenir une peau grasse ou à tendance grasse et/ou un désordre esthétique cutané associé, chez un individu en ayant besoin, comprenant au moins une étape d'administration, audit individu, d'au moins une quantité efficace d'au moins un composé tel que défini selon l'une des revendications 1 à 5.

12. Composé représenté par la formule générale (IIa) : 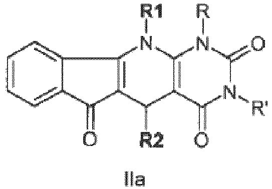 dans laquelle
- R¹ représente H, et
- R² représente un groupe alkyle en C₁-C₄, linéaire ou ramifié, saturé ou insaturé ; un groupe benzyle ; ou 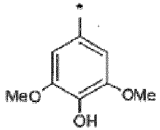
- R et R' représentent un groupe méthyle, ou un sel physiologiquement acceptable de celui-ci.

13. Composition cosmétique ou dermatologique comprenant, dans un milieu physiologiquement acceptable, une quantité efficace d'au moins un composé représenté par la formule générale (IIa) tel que défini selon la revendication 12.
